Europäisches Patentamt

European Patent Office

Office européen des brevets

(19)

(11) **EP 0 707 603 B1**

(12) **EUROPÄISCHE PATENTSCHRIFT**

(45) Veröffentlichungstag und Bekanntmachung des Hinweises auf die Patenterteilung:
**03.09.1997   Patentblatt 1997/36**

(21) Anmeldenummer: **94923693.9**

(22) Anmeldetag: **28.06.1994**

(51) Int Cl.6: **C08F 220/04**, A61L 15/60, A61L 15/24

(86) Internationale Anmeldenummer:
**PCT/EP94/02096**

(87) Internationale Veröffentlichungsnummer:
**WO 95/02002 (19.01.1995 Gazette 1995/04)**

(54) **PULVERFÖRMIGE, VERNETZTE, WÄSSRIGE FLÜSSIGKEITEN SOWIE KÖRPERFLÜSSIGKEITEN ABSORBIERENDE POLYMERE, VERFAHREN ZU IHRER HERSTELLUNG UND IHRE ANWENDUNG**

POWDER-FORM CROSS-LINKED POLYMERS CAPABLE OF ABSORBING AQUEOUS LIQUIDS AND BODY FLUIDS, METHOD OF PREPARING THEM AND THEIR USE

POLYMERES RETICULES, EN POUDRE, ABSORBANT LES LIQUIDES AQUEUX ET LES LIQUIDES CORPORELS, LEUR PROCEDE DE FABRICATION ET LEUR UTILISATION

(84) Benannte Vertragsstaaten:
**AT BE CH DE DK ES FR GB GR IE IT LI LU MC NL PT SE**

(30) Priorität: **09.07.1993  DE 4323001**
**31.05.1994  DE 4418818**

(43) Veröffentlichungstag der Anmeldung:
**24.04.1996   Patentblatt 1996/17**

(73) Patentinhaber: **Stockhausen GmbH & Co. KG**
**47805 Krefeld (DE)**

(72) Erfinder:
• **DAHMEN, Kurt**
**D-41239 Mönchengladbach (DE)**
• **HERRMANN, Edgar**
**D-41334 Nettetal (DE)**

• **PFLÜGER, Klaus**
**D-47807 Krefeld (DE)**

(74) Vertreter: **Klöpsch, Gerald, Dr.-Ing. Patentanwalt**
**Boehmert & Boehmert**
**Anwaltssozietät,**
**Benrather Schlossalle 53**
**40597 Düsseldorf (DE)**

(56) Entgegenhaltungen:
**DE-A- 4 020 780        US-A- 2 833 745**
**US-A- 5 118 719**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft pulverförmige, vernetzte, wässrige Flüssigkeiten sowie Blut absorbierende Polymere (Superabsorber) mit verbesserten Eigenschaften hinsichtlich Aufnahmegeschwindigkeit, Quellung und Rückhaltevermögen von wäßrigen Flüssigkeiten unter hoher Belastung, ein Verfahren zur Herstellung dieser Polymeren sowie ihre Verwendung in absorbierenden Sanitärartikeln, wie Babywindeln, bei der Erwachseneninkontinenz, der Damenhygiene und der Wundabdeckung.

Superabsorber sind wasserunlösliche, vernetzte Polymere, die in der Lage sind, unter Quellung und Ausbildung von Hydrogelen große Mengen an wäßrigen Flüssigkeiten und Körperflüssigkeiten, wie z.B. Urin oder Blut, aufzunehmen und die absorbierte Flüssigkeitsmenge unter einem bestimmten Druck zurückzuhalten. Durch diese charakteristischen Absorptionseigenschaften finden die Polymeren hauptsächlich Anwendung bei der Einarbeitung in Sanitärartikel, wie z.B. in Babywindeln und Damenbinden.

Während in der Entwicklung der Superabsorber zunächst allein das sehr hohe Quellvermögen bei Kontakt mit Flüssigkeit, auch freie Quellkapazität genannt, im Vordergrund stand, hat sich später gezeigt, daß es nicht nur auf die Menge der absorbierten Flüssigkeit ankommt, sondern auch auf die Festigkeit des gequollenen Gels. Absorptionsvermögen oder auch Quellvermögen oder freie Quellkapazität genannt einerseits und Gelfestigkeit bei einem vernetzten Polymeren andererseits stellen jedoch gegenläufige Eigenschaften dar, wie bereits aus der US-Re 32,649 bekannt ist. Das bedeutet, daß Polymere mit besonders hohem Absorptionsvermögen nur eine geringe Festigkeit des gequollenen Gels aufweisen mit der Folge, daß das Gel unter einem angewendeten Druck (z.B. Körperdruck) deformierbar ist und die weitere Flüssigkeitsverteilung und -aufnahme verhindert. Nach der US-Re 32,649 soll daher ein ausgewogenes Verhältnis zwischen Absorptionsvermögen (Gelvolumen) und Gelstärke angestrebt werden, damit bei der Verwendung derartiger Superabsorber in einer Windelkonstruktion Flüssigkeitsaufnahme, Flüssigkeitstransport, Trockenheit der Windel und der Haut gewährleistet sind. Dabei kommt es nicht nur darauf an, daß das Polymer Flüssigkeit unter nachfolgender Einwirkung eines Drucks zurückhalten kann, nachdem das Polymer zunächst frei quellen konnte, sondern auch darauf, Flüssigkeiten auch gegen einen gleichzeitig, d.h. während der Flüssigkeitsabsorption, ausgeübten Druck aufzunehmen, wie es unter praktischen Gegebenheiten geschieht, wenn ein Baby oder eine Person auf einem Sanitärartikel sitzt oder liegt oder wenn es, z.B. durch Beinbewegungen, zur Einwirkung von Scherkräften kommt. Diese spezifische Absorptionseigenschaft wird in der EP 0 339 461 als Aufnahme unter Druck bezeichnet.

Der zunehmenden Tendenz, aus verständlichen ästhetischen Gründen und aus Umweltaspekten (Verringerung des Deponievolumens) die Sanitärartikel immer kleiner und dünner zu gestalten, kann nur dadurch entsprochen werden, daß man den großvolumigen Fluffanteil in Windeln reduziert und gleichzeitig den Anteil an Superabsorber erhöht. Hierdurch muß der Superabsorber zusätzliche Aufgaben bezüglich Flüssigkeitsaufnahme und -transport übernehmen, die vorher der Fluff erfüllte, welche die bisher bekannten Superabsorber nicht ausreichend erfüllen können.

Dies ist besonders dann der Fall, wenn der Anteil des Superabsorbers in der Absorptionszone eines Hygieneartikels auf 40 bis 60 Gew% und darüber hinaus erhöht wird. Hierbei kann es wegen zu geringer Aufnahmegeschwindigkeit des Superabsorbers bei mehreren Miktionen durch eine Blockade im Flüssigkeitstransport und in der Flüssigkeitsverteilung, insbesondere unter Druckbelastung, zum sog. leakage kommen, d.h. die Flüssigkeit wird durch den Hygieneartikel nicht mehr aufgenommen und führt zur Nässung der Windeloberfläche und damit der Haut.

Um superabsorbierende Polymere bereitzustellen, die charakteristische Eigenschaften wie hohe Aufnahmekapazität, hohe Gelstärke und hohes Aufnahmevermögen unter Druck besitzen, ist es notwendig, die Polymerharze nachträglich einer Oberflächenbehandlung zu unterwerfen.

So werden in der US-PS 4 043 952 zur Verbesserung der Dispergierbarkeit in Wasser polyvalente Metallverbindungen und in der US-PS 4 051 086 zur Verbesserung der Aufnahmegeschwindigkeit Glyoxal empfohlen. Die DE-OS 27 40 169 beschreibt die Herstellung von Absorptionsmitteln auf der Basis von Kalium- und Ammonium-acrylathaltigen Polymerisaten, die mit Polyolen behandelt und als Pulver und Folien in Windeln und anderen Hygiene- und medizinischen Artikeln eingesetzt werden. In den Patentschriften EP 0 083 022 (zur verbesserten Dispergierbarkeit in Wasser und zur Verbesserung des Absorptionsvermögens), DE-OS 33 31 644 (zur Verbesserung der Resistenz gegen Salzlösungen bei hoher Wasseraufnahmegeschwindigkeit),
DE-OS 35 07 775 (ebenfalls zur Erhöhung der Salzbeständigkeit bei guter Flüssigkeitsabsorption und Gelfestigkeit), DE-OS 35 23 617 zur Verbesserung der Fließfähigkeit und zum Verhindern des Zusammenbackens, DE-OS 36 28 482 (zur Verbesserung der Wasseraufnahme bei wiederholter Verwendung) und EP 0 349 240 (zur Erzielung eines Gleichgewichtes zwischen Absorptionsvermögen und Absorptionsgeschwindigkeit sowie Gelfestigkeit und Saugkraft) wird die Nachbehandlung von Harzen mit zwei- oder mehrfunktionelle Gruppen enthaltenden Vernetzungsmitteln beschrieben, die mit den Carboxyl- oder Carboxylatgruppen oder anderen im Polymer enthaltenden Gruppen reagieren können. Hierbei wird entweder das Pulver direkt mit den Komponenten, ggf. unter Mitverwendung geringer Mengen Wasser und Lösungsmittel, vermischt oder das Pulver in einem inerten Lösungsmittel dispergiert oder 10 bis 40 Gew% Wasser enthaltende Polymere werden in einem hydrophilen oder hydrophoben Lösungsmittel dispergiert und anschließend oder gleichzeitig mit dem Vernetzungsmittel vermischt. Als Vernetzungsmittel können Polyglycidylether, Haloepoxiverbindungen, Polyole, Polyamine oder Polyisocyanate verwendet werden. Neben diesen werden in DE-OS 33 14 019, EP 0 317 106 und DE-OS 37 37 196 weiterhin polyfunktionelle Aziridinverbindungen, Alkyl-di-(tri)-halogenide und öllösliche Polyepoxiverbindungen erwähnt. In der DE-OS 35 03 458 (zur Erzielung eines Polymeren mit gutem Wasserabsorptionsvermögen, hoher Wasserabsorptionsrate und hoher Gelfestigkeit bei nicht-klebrigem Gel) erfolgt die Aufbringung eines Vernetzungsmittels auf ein Polymerharz in Gegenwart eines inerten anorganischen Pulvermaterials wie $SiO_2$ ohne Verwendung organischer Lösungsmittel. Nach der DE-PS 40 20 780 wird die verbesserte Absorption unter Druck durch oberflächenvernetzende Behandlung eines Polymerharzes mit 0,1 bis 5 Gew% Alkylencarbonat erreicht.

Allen genannten Verfahren ist gemeinsam, daß anschließend eine Temperaturbehandlung der Harze erfolgt.

Die nach diesem Stand der Technik gewonnenen Superabsorber weisen unter einer Druckbelastung von 20 g/$cm^2$ eine hohe Quellfähigkeit aus, die nach der Lehre der DE-PS 40 20 780 bei einer Aufnahme unter diesem Druck (AUL) von 28 bis 34 g/g an 0,9%iger Natriumchloridlösung liegt. Es wird weiter berichtet, daß die nach der Patentschrift hergestellten Produkte eine hohe Anfangsgeschwindigkeit der Flüssigkeitsaufnahme unter Druck aufweisen, so daß 80 % der Gesamtkapazität bereits nach 15 Minuten erreicht werden.

Es hat sich jedoch herausgestellt, daß die relativ hohe Aufnahmegeschwindigkeit bei den bisher durch Nachbehandlung nach dem Stand der Technik herstellbaren Polymerharzen besonders dann gegeben ist, wenn die Flüssigkeitsaufnahme unter gleichzeitig einwirkender Druckbelastung erfolgt. Bei belastungsfreier Quellung ist die Aufnahme jedoch noch verbesserungsbedürftig.

Für die praktische Verwendung von Absorberharzen in Hygieneartikeln ist eine schnelle Flüssigkeitsaufnahme unter belastungsfreier Quellung nämlich sehr wichtig, weil z.B. Kinder- oder Erwachsenenwindeln nicht immer durch das Körpergewicht belastet sind und in diesen Fällen ebenfalls eine schnelle Absorption großer Flüssigkeitsmengen erfolgen muß, um Leakage zu vermeiden.

Die nach dem Stand der Technik bekannten Absorberharze weisen weiterhin den Nachteil auf, daß bei höherer Belastung als 20 g/$cm^2$ die Quellfähigkeit stark zurückgeht. Bei einem danach bekannten Polymer, das unter einer Belastung von 20 g/$cm^2$ eine AUL von 30 g/g besitzt, fällt die AUL bei einer Belastung von 40 g/$cm^2$ auf 15 g/g und bei 60 g/$cm^2$ auf 9 g/g. Dieser Abfall in der Absorptionskapazität unter hohem Druck wirkt sich besonders negativ in den neuen Windelkonstruktionen mit erhöhtem Superabsorberanteil aus, in denen das Absorberharz auch weitgehend den Flüssigkeitstransport zu den entfernteren Speicherzonen übernehmen muß. Hierbei bildet sich infolge der unzureichenden Gelstabilität ein weiches, unter hohem Druck deformierbares Gel, das durch das sog. Gel-blocking den weiteren Flüssigkeitstransport behindert.

Es ist deshalb Aufgabe der vorliegenden Erfindung, superabsorbierende Polymere bereitzustellen, die neben einer verbesserten Absorptionsgeschwindigkeit und hohem Retentionsvermögen in besonderem Maße eine hohe Aufnahme unter erhöhter Druckeinwirkung besitzen.

Die Aufgabe wird gelöst durch ein pulverförmiges wasserquellbares vernetztes, Wasser, wäßrige Flüssigkeiten, insbesondere Körperflüssigkeiten absorbierendes Polymerisat,

gebildet aus

a) 55-99,9 Gew.% polymerisierten, ungesättigten, polymerisierbaren säuregruppenenthaltenden Monomeren, wobei diese Monomeren zu mindest 50 Mol% neutralisiert als Salze vorliegen

b) 0-40 Gew.% polymerisierten, ungesättigten, mit a) copolymerisierbaren Monomeren,

c) 0,1-5,0 Gew.% eines Vernetzungsmittels,

d) 0-30 Gew.% eines wasserlöslichen Polymeren, wobei die Summe a)-d) 100 Gew.% ergibt

und das Polymerisat eine Aufnahemekapazität für eine 0,9%ige NaCl-Lösung von mindestens 12 g/g Polymerisat, vorzugsweise 16 g/g Polymerisat bei einer Belastung von 60 g/$cm^2$ aufweist.

Überraschenderweise hat sich gezeigt, daß superabsorbierende Polymere mit neutralisierten Säuregruppen, deren Kationen ausgewählt sind aus der Gruppe Lithium, Natrium und vorzugsweise Kalium, Rubidium, Cäsium, Ammonium, Monomethylammonium, Dimethylammonium oder Trimethylammonium, eine bedeutende Verbesserung der Absorptionsgeschwindigkeit aufweisen, wenn bei der Herstellung der Polymerprodukte vor dem Polymerisationsschritt ein Treibmittel auf der Basis von Kohlendioxid der Monomerlösung zugesetzt worden ist. Gleichzeitig weisen die erfindungsgemäßen Superabsorber eine Verbesserung der Absorption unter hoher Druckeinwirkung dadurch auf, daß die teilchenförmigen Absorberharze mit einem reaktiven, mehrfunktionellen Nachvernetzungsmittel behandelt und anschließend auf 120 - 300°C erhitzt worden sind.

In der US-PS 4 529 739 werden Absorptionsmittel beschrieben die ausgehend von hydrophoben Polymerisaten in Latexform durch Verseifung und unter Zusatz von Carbonaten als Blähmittel hergestellt werden.

Aus der US-PS 5 118 719 ist bekannt, superabsorbierende Polymere mit verbesserter Wasserabsorptionsrate mittels carbonathaltiger Blähmittel herzustellen, die durch Freisetzung von Kohlendioxid zu einem Hydrogel mit mikrozellularer Struktur führen. Wie aus den Beispielen der US-PS 5 118 719 hervorgeht, ist zwar die Absorptionsrate verbessert, es ist allerdings ein Abfall in der Absorptionskapazität festzustellen. Absorberharze, hergestellt nach der US-PS 5 118 719, zeigen eine wesentlich schlechtere Aufnahme unter Druck (AUL) als sie die Produkte nach dem Stand der Technik, z.B. nach der DE-PS 40 20 780, aufweisen. Nach den bekannten Verfahrensweisen werden insbesondere keine wasserabsorbierenden Harze erhalten, die eine Verbesserung der Absorptionsgeschwindigkeit bei belastungsfreier Quellung aufweisen, obwohl sie eine hohe Aufnahme unter einem Druck von 20 g/cm$^2$ besitzen. Somit war aus dem vorstehend beschriebenen Stand der Technik kein Hinweis ableitbar, wie die Eigenschaftskombinationen aus verbesserter Aufnahmegeschwindigkeit bei gleichzeitig belastungsfreier sowie belasteter Aufnahme und verbessertem Aufnahmevermögen unter hohem Druck erreicht werden kann.

Es wurde nun gefunden, daß verbesserte wasserabsorbierende Harze erhalten werden, wenn Hydrogele mit mikrozellularer Struktur, die unter Verwendung von Blähmitteln erzeugt werden und diese Harze einer oberflächenvernetzenden Behandlung, z.B. nach dem Verfahren der DE-PS 40 20 780 unterworfen werden. Weiterhin wurde gefunden, daß die Kationen von Lithium, Natrium und vorzugsweise von Kalium, Rubidium, Cäsium sowie Ammonium, Monomethylammonium, Dimethylammonium oder Trimethylammonium, die in den bei der Neutralisation der säuregruppenenthaltenden Monomeren entstehenden Salzen vorliegen, einen bestimmenden Einfluß auf die Absorptionskapazität und -geschwindigkeit ausüben. Hierbei zeigte sich, daß eine deutliche Verbesserung der Aufnahmekapazität unter Gewichtsbelastung sowie eine Verbesserung der Aufnahmegeschwindigkeit eintritt.

Bevorzugt zu verwendende Kationen sind das Kaliumion und das Ammoniumion.

Wasserabsorbierende Harze die zur oberflächenvernetzenden Behandlung verwendet werden können, werden erhalten durch Polymerisation von 55 - 99,9 Gew% Monomeren mit Säuregruppen, wie z.B. Acrylsäure, Methacrylsäure, 2-Acrylamido-2-methylpropansulfonsäure oder Mischungen dieser Monomeren; die Säuregruppen liegen mindestens zu 50 Mol% neutralisiert vor. Besonders bevorzugt ist ein Harz, das aus vernetzter Acrylsäure oder Methacrylsäure gebildet ist, die zu 50 - 80 Mol% neutralisiert ist.

Die Neutralisation der Säuregruppen in den wässrigen Monomerlösungen erfolgt mit Basen nach bekannten Methoden, z.B. mit Laugen, Carbonaten oder Aminen. Bevorzugte Neutralisationsmittel sind neben Natriumlauge vor allem Kalilauge und Ammoniak. Als weitere Monomere können für die Herstellung der wasserabsorbierenden Harze 0 - 40 Gew% Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Höhere Anteile als 40 % dieser Monomerer verschlechtern die Quellfähigkeit der Harze.

Als Vernetzer können alle Verbindungen verwendet werden, die mindestens zwei ethylenisch ungesättigte Doppelbindungen oder eine ethylenisch ungesättigte Doppelbindung und eine gegenüber Säuregruppen reaktive funktionelle Gruppe oder mehrere gegenüber Säuregruppen reaktive funktionelle Gruppen tragen. Beispielhaft seien genannt: Acrylate und Methacrylate von Polyolen, wie Butandiol-diacrylat, Hexandiol-dimethacrylat, Polyglykol-diacrylat, Trimethylolpropantriacrylat, oder Allylacrylat, Diallylacrylamid, Triallylamin, Diallylether, Methylenbisacrylamid oder N-methylolacrylamid, weiter Polyglycidylether, wie z.B. Ethylenglykoldiglycidylether und Glycerinpolyglycidylether und/oder Polyole, wie Glycerin, Trimethylolpropan und/oder Polyalkylenglykole, wie Polyethylenglykol 200 bis 600. Ebenso können Polyamine verwendet werden.

Als wasserlösliche Polymere können im wasserabsorbierenden Harz 0 - 30 Gew% teiloder vollverseifter Polyvinylalkohol, Polyvinylpyrrolidon, Stärke oder Stärkederivate, Polyglykole oder Polyacrylsäuren enthalten sein. Das Molekulargewicht dieser Polymeren ist unkritisch, solange sie wasserlöslich sind. Bevorzugte wasserlösliche Polymere sind Stärke oder Polyvinylalkohol oder Gemische dieser Polymeren. Der bevorzugte Gehalt an solchen wasserlöslichen Polymeren im wasserabsorbierenden Harz liegt bei 1 - 5 Gew%, insbesondere wenn Stärke und/oder Polyvinylalkohol als lösliche Polymere vorhanden sind. Die wasserlöslichen Polymere können als Pfropfpolymere mit den Säuregruppen enthaltenden Polymeren vorliegen.

Bevorzugt werden neben Harzen, die durch vernetzende Polymerisation teilneutralisierter Acrylsäure erhalten worden sind, solche verwendet, die zusätzlich Anteile von pfropfpolymerisierter Stärke oder von Polyvinylalkohol enthalten.

Die Herstellung der erfindungsgemäßen Absorberharze erfolgt nach bekannten Verfahren. Das kann sowohl nach dem Prinzip der wässrigen Lösungspolymerisation (Gelverfahren) als auch nach dem inversen Emulsions-/Suspensionsverfahren erfolgen.

Erfindungsgemäß muß in der Monomerlösung oder -dispersion ein Treibmittel auf der Basis von Kohlenstoffdioxid als Carbonat oder in Form von Kohlenstoffdioxid, gasförmig oder fest, gelöst oder dispergiert werden. Als Carbonate können eine Menge von 0,1-5,0 Gew.%, bezogen auf wasserfreie Polymersubstanz, z.B. Natriumcarbonat, Natriumhydrogencarbonat, Kaliumcarbonat, Kaliumhydrogencarbonat, Ammoniumcarbonat, Magnesiumcarbonat, Calciumcarbonat oder Gemische dieser Substanzen verwendet werden. Bei der Verwendung von festem Kohlenstoffdioxid (Trockeneis) kann gleichzeitig die Kühlung der Monomerenlösung oder -dispersion auf Temperaturen zwischen - 10°C und 30°C bevorzugt zwischen 0 und 10°C sowie die Entfernung von Sauerstoff erfolgen.

Bei der oberflächenvernetzenden Behandlung gibt es hinsichtlich der Teilchenform des eingesetzten Absorberharzes keine speziellen Einschränkungen. Das Polymer kann in Form von Kügelchen vorliegen, die durch inverse Suspensionspolymerisation erhalten wurden oder in Form von unregelmäßig geformten Teilchen, die durch Trocknung und Zerkleinerung der Masse aus der Lösungspolymerisation stammen. Die Trocknung des Hydrogels erfolgt bei Temperaturen von 80-200°C insbesondere bei 100-180°C und vorzugsweise bei 120-150°C. Die Teilchengröße liegt normalerweise zwischen 20 und 3000 µm, bevorzugt zwischen 50 und 1000 µm.

Zur oberflächenvernetzenden Behandlung können die wasserabsorbierenden Harze mit den bekannten Nachvernetzungsmitteln auf Basis mindestens bifunktioneller, mit Säuregruppen, insbesondere Carboxylgruppen reaktionsfähigen Verbindungen verwendet werden. Die wasserabsorbierenden Harze können mit den Nachvernetzungsmitteln direkt, in wäßrig alkoholischer Lösung oder in wäßriger Lösung vermischt werden. Die Menge des Nachvernetzungsmittels beträgt 0,01 bis 10 Gew.-%, vorzugsweise 0,01 bis 4,0 Gew.-% und besonders bevorzugt 0,01 bis 2,0 Gew.-%, bezogen auf das wasserabsorbierende Harz. Auch Mischungen verschiedener Nachvernetzungsmittel können verwendet werden. Die Menge Alkohol wird von der Löslichkeit des Mittels bestimmt und wird aus technischen Gründen, wie z.B. Explosionsschutz, so gering wie möglich gehalten. Geeignete Alkohole sind Methanol, Ethanol, Butanol oder Butylglykol sowie Gemische dieser Alkohole. Bevorzugtes Lösungsmittel ist Wasser, das in einer Menge von 0,3 - 5,0 Gew% , bezogen auf Harz, verwendet wird. Es besteht auch die Möglichkeit, das Nachvernetzungsmittel aus einer Pulvermischung, z.B. mit einem anorganischen Trägermaterial wie $SiO_2$, aufzubringen.

Als oberflächenvernetzende, mindestens zweifunktionelle, mit Säuregruppen reaktionsfähigen Verbindungen werden Polyole wie Glycerin und/oder Polyalkylenglykole wie Polyethylenglykol und/oder Polyamine wie Triethanolamin bevorzug. Besonders bevorzugt sind als Oberflächenvernetzungsmittel Alkylencarbonate gemäß DE-PS 40 20 780.

Um die gewünschten Eigenschaften zu erzielen, ist eine gleichmäßige Verteilung des Mittels auf dem Harzpulver erforderlich. Dazu führt man die Vermischung in geeigneten Mischern durch, wie z.B. Wirbelbettmischer, Schaufelmischer, Walzenmischer oder Doppelschneckenmischer.

Es besteht auch die Möglichkeit, die Behandlung des Absorberharzes während eines der Verfahrensschritte bei der Herstellung des Polymerharzes vorzunehmen. Hierzu ist besonders der Prozeß der inversen Suspensionspolymerisation geeignet.

Die sich an die Zugabe des Oberflächenvernetzungsmittels anschließende thermische Behandlung wird bei 100 - 300°C durchgeführt, bevorzugt bei 120 - 250°C. Sie ist abhängig von der Verweilzeit und der Art des Reaktionsmittels. Bei 150°C muß die thermische Behandlung über mehrere Stunden durchgeführt werden, während bei 250°C wenige Minuten, z.B. 0,5 bis 5 Minuten ausreichen, um die gewünschten Eigenschaften zu erzielen. Die thermische Behandlung kann in üblichen Trocknern oder Öfen durchgeführt werden; beispielhaft seien Drehrohröfen, Wirbelbetttrockner, Paddeltrockner, Tellertrockner oder Infrarottrockner genannt.

Die Polymeren gemäß der Erfindung können in großtechnischer Weise nach kontinuierlichen und diskontinuierlichen Verfahren hergestellt werden. Die erfindungsgemäßen Mittel können für weite Anwendungsgebiete eingesetzt werden. Wenn sie z.B. als Absorbierungsmittel in Damenbinden und Windeln oder zur Wundabdeckung verwendet werden, dann besitzen sie die Eigenschaft, daß sie große Mengen an Menstruationsblut, Urin oder anderen Körperflüssigkeiten schnell absorbieren. Die Absorptionsfähigkeit und -geschwindigkeit ist viel höher als bei bekannten Produkten. Da die erfindungsgemäßen Mittel die absorbierten Flüssigkeiten unter hohem Druck aufnehmen und zurückhalten, sind die Mittel besonders anwendungsfreundlich. Sie sind bevorzugt geeignet, in höheren Konzentrationen, bezogen auf hydrophiles Fasermaterial wie z.B. Fluff, eingesetzt zu werden, als dies bisher möglich war.

Bei der Verwendung kann der Anteil des absorbierenden Polymerisats über 35 Gew.% liegen, 15-100 Gew.% und vorzugsweise 30 - 70 Gew.°% bezogen auf die vom absorbierenden Polymerisat mit dem Fluff gebildete Menge betragen. Die erfindungsgemäßen Polymerisate zeichnen sich dabei durch eine verbesserte Verteilung der Flüssigkeit, insbesondere in den überwiegend Polymerisat enthaltenden Schichten direkt nach der Flüssigkeitsaufnahme aus.

Die erfindungsgemäßen Polymerisate sind weiterhin als Absorptionsmittel für Wasser und wässrige Flüssigkeiten geeignet zur Verwendung in strom- und lichtleitenden Kabeln, in Verpackungsmitteln sowie als Bodenverbesserungsmittel und als künstlicher Boden zur Pflanzenzüchtung.

Testmethoden:

Zur Charakterisierung der wasserabsorbierenden Harze wurden Retention (TB), Aufnahme unter Druck (AUL) und Aufnahmegeschwindigkeit (AV bzw. Vortex) gemessen.

Die Retention wird nach der Teebeutelmethode bestimmt und als Mittelwert aus drei Messungen angegeben. Ca. 200 mg Harz werden in einen Teebeutel eingeschweißt und für 20 Minuten in 0,9%ige NaCl-Lösung getaucht. Anschließend wird der Teebeutel in einer Schleuder (23 cm Durchmesser, 1.400 Upm) 5 Minuten geschleudert und gewogen. Einen Teebeutel ohne wasserabsorbierendes Harz läßt man als Blindwert mitlaufen:

$$Retention = \frac{Auswaage - Blindwert}{Einwaage} \ [g/g]$$

Die Aufnahme unter Druck (AUL) wird nach der in der EP 0 339 461, Seite 7, beschriebenen Methode bestimmt: In einen Zylinder mit Siebboden gibt man die Einwaage an Superabsorber und belastet das Pulver mit einem Stempel, der einen Druck von 20 g/cm$^2$ bzw. 40 g/cm$^2$ bzw. 60 g/cm$^2$ ausübt. Der Zylinder wird anschließend auf einen Demand-Absorbency-Tester (DAT) gestellt, wo man den Superabsorber eine Stunde lang 0,9%ige NaCl-Lösung saugen läßt.

Die Absorptionsgeschwindigkeit (AV) wird in abgewandelter Form nach der Methode Aufnahme unter Druck (AUL), wie in der EO 0 339 461 auf Seite 7 beschrieben, bestimmt. Die Aufnahme der 0,9%igen Kochsalzlösung wird in diesem Fall ohne Belastung des Pulvers mit zusätzlichen Gewichten gemessen. Nach 15 s, 30 s, 1, 3, 5, 10 und 15 min wird die aufgenommene Flüssigkeitsmenge durch Auswiegen bestimmt. Die Messung wird als Dreifachbestimmung durchgeführt.

Der Vortex Test zur Bestimmung der Absorptionsgeschwindigkeit wird in Anlehnung an die in der WO 87/03208 auf Seite 9 beschriebenen Methode durchgeführt. Die Einwaage beträgt 2,0 g Polymer, und es wird die Zeit vom Einstreuen der Polymers in den Flüssigkeitskegel bis zum Verschwinden des Vortex in Sekunden gemessen.

## Beispiele

### Vergleichsbeispiel 1

Nach Beispiel 4 der DE-PS 40 20 780 wird eine wässrige Monomerlösung, bestehend aus einer Mischung von Natriumacrylat und Acrylsäure im Molverhältnis 70:30 und Triallylamin als Vernetzer polymerisiert. Das entstandene Gel wird zerkleinert, getrocknet, gemahlen und auf einen Kornbereich von 90 - 850 μm abgesiebt.

Das pulverförmige Produkt wird mit einer Mischung aus 0,2/1,0/2,0 % 1,3-Dioxolan-2-on/Wasser/Ethanol, bezogen auf Pulver, vermischt und 1 Stunde auf 180°C erhitzt.

Produkteigenschaften in Tabelle 1.

### Vergleichsbeispiel 2

Nach Beispiel 4 der US-PS 5.118.719 wird eine wässrige Monomerlösung, bestehend aus einer Mischung von Natriumacrylat und Acrylsäure im Molverhältnis 70:30 und Triallylamin als Vernetzer, nach Zugabe von 0,5 % basischem Magnesiumcarbonat polymerisiert. Das entstandene Gel wird zerkleinert, getrocknet, gemahlen und abgesiebt (90 - 850 μm).

Tabelle 1

| Vergleichsbeispiel | 1 | 2 |
|---|---|---|
| TB | 30 | 26 g/g |
| AUL 20 g/cm$^2$ | 30 | 20 g/g |
| 40 g/cm$^2$ | 15 | 9 g/g |
| 60 g/cm$^2$ | 9 | 8 g/g |
| Vortex | 83 | 18 s |
| AV 15 s | 2 | 7 g/g |
| 30 s | 4 | 15 g/g |
| 1 min | 9 | 24 g/g |
| 3 min | 19 | 33 g/g |
| 5 min | 25 | 36 g/g |
| 10 min | 34 | 37 g/g |
| 15 min | 39 | 37 g/g |
| TB = Teebeuteltest AUL = Aufnahme unter Druck (absoption under load) AV = Absorptionsgeschwindigkeit (absorption velocity) | | |

Beispiele 1-7

Eine wäßrige Monomerlösung bestehend aus einer Mischung aus

Beispiel 1: Kaliumacrylat
Beispiel 2: Kaliumacrylat
Beispiel 3: Kaliumacrylat
Beispiel 4 : Kaliumacrylat
Beispiel 5: Ammoniumacrylat
Beispiel 6: Methylammoniumacrylat

und Acrylsäure im Molverhältnis 70/30 und Triallylamin als Vernetzer wurde unter Zusatz eines Carbonats und 0-1 % Polyvinylalkohol (PVA) polymerisiert. Das entstandene Gel wurde zerkleinert, getrocknet, gemahlen und auf 90-850µm abgesiebt.

Das pulverförmige gesiebte Polymer wurde mit einer Mischung aus 1,3-Dioxolan-2-on bzw. Ethylenglykoldiglycidylether (EGDE),Wasser und Ethanol vermischt und 0,5-1 h auf 120-300 °C erhitzt.

Beispiele 7 und 8

Eine wäßrige Monomerlösung bestehend aus einer Mischung aus

Beispiel 7: Kaliumacrylat
Beispiel 8: Ammoniumacrylat

und Acrylsäure im Molverhältnis 70/30 und Triallylamin als Vernetzer unter Zusatz von 0-1 % Polyvinylalkohol (PVA) wurde mit Kohlendioxid (durch Eintrag von Trockeneis) gesättigt und anschließend polymerisiert. Das entstandene Gel wurde zerkleinert, getrocknet, gemahlen und auf 90-850µm abgesiebt.

Das pulverförmige gesiebte Polymer wurde mit einer Mischung aus 1,3-Dioxolan-2-on bzw. Ethylenglykoldiglycidylether (EGDE),Wasser und Ethanol vermischt und 0,5-1 h auf 120-300 °C erhitzt.

Zusammensetzung, Reaktionsbedingungen und Produkteigenschaften sind in Tabelle 2 zusammengefaßt.

Tabelle 2

| Beispiel | 1 | 2 | 3 | 4 | |
|---|---|---|---|---|---|
| Kation | K | K | K | K | |
| Vernetzer [%] | 0,4 | 0,4 | 0,4 | 0,5 | |
| Carbonat [%] | 1,5 Kalium | 2,5 Kalium | 1,5 Kalium | 0,5Kalium | |
| PVA [%] | 1,0 | 1,0 | - | 1,0 | |
| 1,3-Dioxolan-2-on [%] | 0,5 | 0,5 | 0,5 | 0,5 | |
| Wasser [%] | 2,0 | 2,0 | 2,0 | 2,0 | |
| Ethanol [%] | 4,0 | 2,0 | 2,0 | 2,0 | |
| Zeit[min] | 30 | 30 | 30 | 30 | |
| Temperatur [C] | 200 | 180 | 200 | 200 | |
| TB | 27 | 28 | 27 | 27 | g/g |
| AUL 20 g/cm$^2$ | 25 | 28 | 27 | 25 | g/g |
| 40 g/cm$^2$ | 20 | 20 | 22 | 23 | g/g |
| 60 g/cm$^2$ | 18 | 14 | 18 | 21 | g/g |
| Vortex | 21 | 16 | 21 | 23 | s |
| AV  15 s | 12 | 10 | 10 | 9 | g/g |
| 30 s | 19 | 20 | 16 | 16 | g/g |
| 1 min | 26 | 28 | 24 | 23 | g/g |
| 3 min | 36 | 38 | 31 | 33 | g/g |
| 5 min | 38 | 43 | 33 | 35 | g/g |
| 10 min | 39 | 44 | 35 | 36 | g/g |
| 15 | 40 | 45 | 37 | 38 | g/g |

Tabelle 2  Fortsetzung

| Beispiel | 5 | 6 | 7 | 8 |
|---|---|---|---|---|
| Kation | $NH_4$ | $NH_3CH_3$ | K | $NH_4$ |
| Vernetzer [%] | 0,3 | 0,3 | 0,3 | 0,3 |
| Carbonat | 1,5 $NH_4^+$ | 1,5 $NH_4^+$ | $CO_2$ | $CO_2$ |
| Sättigungstemp.°C | - | - | 4 | 13 |
| Polyvinylalkohol | - | - | 1,0 | 1,0 |
| EGDE | 0,25 | - | - | 0,25 |
| 1,3-Dioxolan-2-on | - | 1,0 | 0,5 | - |
| Wasser | 2,0 | 8,0 | 2,0 | 2,0 |
| Ethanol | 2,0 | 8,0 | 2,0 | 2,0 |
| Zeit[min] | 60 | 60 | 30 | 60 |
| Temperatur [°C] | 120 | 140 | 200 | 120 |
| TB | 33 | 35 | 27 | 33g/g |
| AUL 20 g/cm² | 28 | 24 | 25 | 29g/g |
| 40 g/cm² | 22 | 17 | 20 | 23g/g |
| 60 g/cm² | 18 | 12 | 16 | 17g/g |
| Vortex | 27 | 28 | 15 | 12s |
| AV  15 s | 8 | 6 | 12 | 14g/g |
| 30 s | 13 | 12 | 17 | 20g/g |
| 1 min | 19 | 18 | 28 | 27g/g |
| 3 min | 31 | 26 | 36 | 36g/g |
| 5 min | 34 | 29 | 40 | 41g/g |
| 10 min | 40 | 34 | 43 | 44g/g |
| 15 | 42 | 37 | 44 | 45g/g |

Vergleichsbeispiel 3

Vergleichsbeispiel 2 wird wiederholt. Die zur Neutralisation erforderliche Natronlauge wird durch Kalilauge im glei-

EP 0 707 603 B1

chen Molverhältnis ersetzt. Der Monomerlösung wird vor der Polymerisation 1,5 % Kaliumcarbonat zugesetzt.

Vergleichsbeispiel 4

Vergleichsbeispiel 1 wird wiederholt. Die zur Netralisation erforderliche Natronlauge wird durch Kalilauge im gleichen Molverhältnis ersetzt. Das erhaltene pulverförmige Polymerisat wird der gleichen Nachbehandlung wie in Vergleichbeispiel 1 unterworfen.

Vergleichsbeispiel 5

Vergleichsbeispiel 3 wird wiederholt. Die zur Netralisation erforderliche Kalilauge wird durch wäßrige Ammoniaklösung im gleichen Molverhältnis ersetzt. Der Monomerlösung wird vor der Polymerisation 0,5 % Ammoniumcarbonat zugesetzt.

Vergleichsbeispiel 6

Vergleichsbeispiel 4 wird wiederholt. Die zur Teilneutralisation erforderliche Kalilauge wird durch wäßrige Ammoniaklösung im gleichen Molverhältnis ersetzt. Das pulverförmige Produkt wird mit einer Mischung aus 0,25/2,0/2,0 EGDE/Wasser/Aceton, bezogen auf Pulver, vermischt und 1 h auf 120°C erhitzt.

| Vergleichsbeispiel | | 3 | 4 | 5 | 6 | |
|---|---|---|---|---|---|---|
| TB | | 31 | 30 | 57 | 54 | g/g |
| AUL | 20g/cm² | 10 | 24 | 8 | 9 | g/g |
| | 40g/cm² | 9 | 16 | 7 | 8 | g/g |
| | 60g/cm² | 7 | 11 | 6 | 8 | g/g |
| Vortex | | 70 | 24 | 12 | 20 | s |
| AV | 15 s | 3 | 8 | 2 | 8 | g/g |
| | 30 s | 7 | 14 | 3 | 12 | g/g |
| | 1 min | 13 | 22 | 3 | 17 | g/g |
| | 3 min | 22 | 32 | 5 | 22 | g/g |
| | 5 min | 29 | 38 | 7 | 29 | g/g |
| | 10 min | 34 | 40 | 9 | 31 | g/g |
| | 15 min | 36 | 42 | 10 | 34 | g/g |

Vergleichsbeispiel 7

Das Polymer aus Beispiel 2 wird vor der Nachbehandlung auf die Fraktion 100 - 300 µm abgesiebt und nicht nachbehandelt. Die Quellgeschwindigkeit (Quellhöhe nach 1 min) und die Quellhöhe nach 10 min wurden mit dem sog. FIRET-Test bestimmt, Methode der Firma Lantor B.V.; Veenendaal/NL (s. Tabelle). Der Test wird so modifiziert, daß anstelle des Tapes 0,2 g Superabsorber auf den Boden des Meßtopfes gleichmäßig verteilt werden und mit einem Vlies oder Tissue abgedeckt werden (simuliertes Tape).

Beispiel 9

Das Polymer aus Beispiel 1 wird vor der Nachbehandlung auf die Fraktion 100 - 300 µm abgesiebt und anschließend der gleichen Nachbehandlung wie in Beispiel 1 unterworfen. Die Prüfung der Produkteigenschaften erfogt wie in Vergleichsbeispiel 7 (s. Tabelle).

Beispiel 10

Das Polymer aus Beispiel 1 wird nach der Nachbehandlung auf die Fraktion 100 - 300 µm abgesiebt. Die Prüfung der Produkteigenschaften erfolgt wie in Vergleichsbeispiel 7 (s. Tabelle).

| Vergleichsbeispiel | 7 | Beispiel 9 | Beispiel 10 |
|---|---|---|---|
| Quellgeschwindigkeit | 2,2 | 8,2 | 9,1 mm/min |
| Quellhöhe nach 10 | 11,8 | 12 | 12 mm |

Die nach den Beispielen 9 und 10 erhaltenen erfindungsgemäßen Produkte sind besonders als Komponete in strom- und lichtleitenden Kabeln zur Abdichtung gegen eindringendes Wasser geeignet.

**Patentansprüche**

1. Pulverförmiges, wasserquellbares, vernetztes, Wasser, wäßrige Flüssigkeiten, insbesondere Körperflüssigkeiten absorbierendes Polymerisat, gebildet aus:

   a) 55 - 99,9 Gew.%, ungesättigten, polymerisierbaren, säuregruppenenthaltenden Monomeren, wobei diese Monomere zu mindestens 50 Mol% neutralisiert als Salze vorliegen
   b) 0 - 40 Gew.% ungesättigten, mit a) copolymerisierbaren Monomeren,
   c) 0,1 - 5,0 Gew.% mindestens eines Vernetzungsmittels,
   d) 0 - 30 Gew.% eines wasserlöslichen Polymeren, wobei die Summe a) - d) 100 Gew.% ergibt,

   dadurch gekennzeichnet, daß das Polymerisat eine Aufnahmekapazität für 0,9%ige NaCl-Lösung von mindestens 12 g/g Polymerisat, vorzugsweise 16 g/g Polymerisat bei einer Belastung von 60 g/cm$^2$ aufweist.

2. Polymerisat nach Anspruch 1, dadurch gekennzeichnet, daß es eine Aufnahmegeschwindigkeit für 0,9%ige NaCl-Lösung von mindestens 10 g/g Polymerisat, vorzugsweise 16 g/g Polymerisat in 30 Sekunden aufweist.

3. Polymerisate nach Anspruch 1 oder 2, gekennzeichnet durch eine Korngröße von 20 bis 3000 µm, vorzugsweise von 100 bis 1000 µm.

4. Pulverförmiges, wasserquellbares, vernetztes, Wasser, wäßrige Flüssigkeiten, insbesondere Körperflüssigkeiten absorbierendes Polymerisat, gebildet aus:

   a) 55 - 99,9 Gew.% ungesättigten, polymerisierbaren, säuregruppenenthaltenden Monomeren, wobei diese Monomere zu mindestens 50 Mol% neutralisiert als Salze vorliegen
   b) 0 - 40 Gew% ungesättigten, mit a) copolymerisierbaren Monomeren,
   c) 0,1 - 5,0 Gew.% mindestens eines Vernetzungsmittels,
   d) 0 - 30 Gew.% eines wasserlöslichen Polymeren, wobei die Summe a) - d) 100 Gew.% ergibt,

   dadurch gekennzeichnet, daß die Salze nach a) als Kationen Li$^+$, Na$^+$, vorzugsweise K$^+$, Cs$^+$, Rb$^+$ und Ammonium-Ionen sowie primäre, sekundäre und tertiäre Methylammonium-Ionen allein oder in Kombination enthalten, die Monomeren vor der Polymerisation mit 0,1 - 5,0 Gew.%, bezogen auf das Polymerisat, eines Treibmittels auf Basis von Kohlendioxid versetzt worden sind, das Polymerisat getrocknet und mit einer oder mehreren zur nachträglichen Oberflächenvernetzung reaktionsfähigen Verbindungen oder deren Lösung oder Dispersion versetzt und auf eine Temperatur von 100 - 300 °C erhitzt worden ist.

5. Polymerisate nach Ansprüchen 1 bis 4, dadurch gekennzeichnet, daß die Säuregruppen enthaltenden Monomeren a)
   aus der Gruppe ausgewählt sind, die aus Acrylsäure, Methacrylsäure, Acrylamidomethylpropansulfonsäure und Mischungen dieser Monomeren besteht
   die Monomeren b)
   aus der Gruppe ausgewählt sind, die aus Acrylamid, Methacrylamid, Hydroxyalkylacrylat, Dimethylaminoalkyl(meth)acrylat, Dimethylaminopropyl(meth)acrylamid, den quaternären Amin- und Ammoniumsalzen dieser vorstehend genannten Monomeren und Mischungen dieser Monomeren besteht,
das Vernetzungsmittel c)
aus der Gruppe ausgewählt ist, die aus Alkylenbisacrylamid, N-Methylolacrylamid, Butandioldiacrylat, Hexandiol-dimethacrylat, Polyglykol-diacrylat, Trimethylolpropantriacrylat, Allylacrylat, Diallylacrylamid, Triallylamin, Diallylether, Polyglycidylether wie Ethylenglykoldiglycidylether und Glycerinpolyglycidylether, Polyole wie Glycerin, Trimethylolpropan, Polyalkylenglykole wie Polyethylenglykol 200 bis 600 und Polyamine oder Mischungen dieser Vernetzungsmittel besteht, das Treibmittel auf Basis von Kohlendioxid, ein Carbonat aus der aus $Na_2CO_3$, $K_2CO_3$, $(NH_4)_2CO_3$, $MgCO_3$, $CaCO_3$, $NaHCO_3$, $KHCO_3$, Mischungen der vorstehenden Carbonate oder Kohlendioxid in gasförmiger oder fester Form bestehenden Gruppen ausgewählt ist.

6. Verfahren zur Herstellung der Polymeren nach den Ansprüchen 1 bis 5 durch Polymerisation der Monomeren a), der mit a) copolymerisierbaren Monomeren b) und des Vernetzungsmittels c) in Gegenwart des wasserlöslichen Polymeren d), dadurch gekennzeichnet, daß die Salze nach a) aus der Gruppe ausgewählt sind, die aus Lithium, Natrium und vorzugsweise Kalium-, Cäsium-, Rubidium-, Ammonium-, primären, sekundären und tertiären Methylammonium-Ionen oder Mischungen dieser Ionen besteht, die Monomeren vor der Polymerisation mit 0,1 - 5,0 Gew.%, bezogen auf das Polymerisat, eines Treibmittels auf Basis von Kohlendioxid versetzt werden, Polymerisation der erhaltenen Monomerenmischung unter Zugabe von radikalbildenden Initiatoren oder Initiierung durch Belichtung oder Bestrahlung unter Ausbildung eines Hydrogels, das Polymerisat getrocknet und mit einer oder mehreren zur nachträglichen oberflächennahen Vernetzung reaktionsfähigen Verbindungen oder deren Lösung oder Dispersion versetzt und auf eine Temperatur von 100 - 300 °C erhitzt wird.

7. Verfahren nach Anspruch 6, dadurch gekennzeichnet, daß das Polymerisat bei Temperaturen von 80 bis 200 °C, insbesondere von 100 bis 180 °C und vorzugsweise von 120 bis 150 °C getrocknet wird.

8. Verfahren nach Anspruch 6, dadurch gekennzeichnet, daß das Polymerisat nach der Trocknung gemahlen wird.

9. Verfahren nach Anspruch 7 oder 8, dadurch gekennzeichnet, daß das Polymerisat auf eine Korngröße von 20 - 3000 μm, vorzugsweise von 50 - 1000 μm abgesiebt wird.

10. Verfahren nach Anspruch 6, dadurch gekennzeichnet, daß zur oberflächennahen Vernetzung auf eine Temperatur von 100 bis 300 °C erhitzt wird.

11. Verfahren nach Anspruch 6, dadurch gekennzeichnet, daß die Polymerisation in wäßriger Lösung durchgeführt wird.

12. Verfahren nach Anspruch 6, dadurch gekennzeichnet, daß die Polymerisation in einer W/O-Dispersion durchgeführt wird.

13. Verwendung der Polymerisate nach den Ansprüchen 1 bis 5 als Komponente in Körperflüssigkeiten absorbierenden Sanitärartikeln und in Wundabdeckungen.

14. Verwendung der Polymerisate nach den Ansprüchen 1 bis 5 als Komponente in Windeln, Damenbinden und Inkontinenzartikeln.

15. Verwendung der Polymerisate nach den Ansprüchen 1 bis 5 in Windeln mit einem Gewichtsanteil der Polymerisate nach den Ansprüchen 1 bis 5, bezogen auf die Gewichtsmenge von Polymerisat und Fluff von 15 - 100 Gew.%, vorzugsweise 35 - 100 Gew.% und besonders bevorzugt von 30 bis 70 Gew.%.

16. Verwendung der Polymerisate nach den Ansprüchen 1 bis 5 als Komponente in strom- und lichtleitenden Kabeln.

17. Verwendung der Polymerisate nach den Ansprüchen 1 bis 5 als Bodenverbesserungsmittel.

18. Verwendung der Polymerisate nach den Ansrpüchen 1 bis 5 als künstlicher Boden zur Pflanzenzüchtung.

19. Verwendung der Polymerisate nach den Ansprüchen 1 bis 5 als Komponente in Verpackungsmitteln.

**Revendications**

1. Polymère pulvérulent, gonflable à l'eau, réticulé, absorbant l'eau, les liquides aqueux, plus particulièrement les liquides corporels, constitué de

   a) 55 à 99,9% en poids de monomères contenant des radicaux acide, insaturés, polymérisables, où ces monomères se présentent pour au moins 50% molaires neutralisés sous forme de sel,
   b) 0 à 40% en poids de monomères, insaturés, copolymérisables avec a),
   c) 0,1 à 5,0% en poids d'au moins un agent de réticulation,
   d) 0 à 30% en poids d'un polymère soluble dans l'eau, où la somme a)-d) est égale à 100% en poids,

   caractérisé en ce que le polymère possède un pouvoir absorbant pour une solution à 0,9% de NaCl d'au moins 12 g/g de polymère, de préférence, de 16g/g de polymère sous une charge de 60 g/cm$^2$.

2. Polymère suivant la revendication 1, caractérisé en ce qu'il présente une vitesse d'absorption pour une solution à 0,9% de NaCl d'au moins 10 g/g de polymère, de préférence, de 16 g/g de polymère en l'espace de 30 secondes.

3. Polymères suivant la revendication 1 ou 2, caractérisés par une granulométrie de 20 à 3000 mm, de préférence, de 100 à 1000 mm.

4. Polymère pulvérulent, gonflable à l'eau, réticulé, absorbant l'eau, les liquides aqueux, plus particulièrement les liquides corporels, constitué de

   a) 55 à 99,9% en poids de monomères contenant des radicaux acide, insaturés, polymérisables, où ces monomères se présentent pour au moins 50% molaires neutralisés sous forme de sel,
   b) 0 à 40% en poids de monomères, insaturés, copolymérisables avec a),
   c) 0,1 à 5,0% en poids d'au moins un agent de réticulation,
   d) 0 à 30% en poids d'un polymère soluble dans l'eau, où la somme a)-d) est égale à 100% en poids,

   caractérisé en ce que les sels selon a) contiennent, à titre de cations, des ions Li$^+$, Na$^+$, de préférence K$^+$, Cs$^+$, Rb$^+$ et ammonium, ainsi que des ions méthylammonium primaire, secondaire et tertiaire, seuls ou en combinaison, on ajoute aux monomères, avant la polymérisation, de 0,1 à 5,0% en poids, par rapport au polymère, d'un agent porogène à base de dioxyde de carbone, on sèche le polymère et on y ajoute un ou plusieurs composés réactifs en vue d'une réticulation superficielle subséquente, ou à sa solution ou sa dispersion et on le chauffe jusqu'à une température de 100 à 300°C.

5. Polymères suivant les revendications 1 à 4, caractérisés en ce que les monomères a) contenant les radicaux acide sont choisis dans le groupe formé par l'acide acrylique, l'acide méthacrylique, l'acide acrylamidométhylpropanesulfonique et les mélanges de ces monomères,
   les monomères b)
   sont choisis dans le groupe formé par l'acrylamide, le méthacrylamide, un acrylate d'hydroxyalkyle, un (méth) acrylate de diméthylaminoalkyle, le diméthylaminopropyl(méth)acrylamide, les sels d'amine et d'ammonium quaternaire des monomères susmentionnés et les mélanges de ces monomères,
   l'agent de réticulation c)
   est choisi dans le groupe formé par un alkylène-bisacrylamide, le N-méthylolacrylamide, le diacrylate de butanediol, le diméthacrylate d'hexanediol, le diacrylate de polyglycol, le triacrylate de triméthylolpropane, l'acrylate d'allyle, le diallylacrylamide, la triallylamine, l'éther diallylique, des éthers polyglycidyliques, comme l'éther diglycidylique de l'éthylèneglycol et l'éther polyglycidylique de la glycérine, des polyols, comme la glycérine, le triméthylolpropane, des polyalkylèneglycols, comme le polyéthylèneglycol 200 à 600 et des polyamines, ou des mélanges de ces agents de réticulation,
   l'agent porogène à base de dioxyde de carbone est choisi dans le groupe formé par un carbonate répondant à l'une des formules suivantes : Na$_2$CO$_3$, K$_2$CO$_3$, (NH$_4$)$_2$CO$_3$, MgCO$_3$, CaCO$_3$, NaHCO$_3$, KHCO$_3$, les mélanges des carbonates précités ou le dioxyde de carbone, sous forme gazeuse ou sous forme solide.

6. Procédé de préparation des polymères suivant l'une quelconque des revendications 1 à 5, par la polymérisation des monomères a), des monomères b) copolymérisables avec a) et de l'agent de réticulation c) en présence du polymère soluble dans l'eau d), caractérisé en ce que les sels selon a) sont choisis dans le groupe formé par les ions lithium, sodium et, de préférence, potassium, césium, rubidium, ammonium, méthylammonium primaire, secondaire et tertiaire, ou des mélanges de ces ions, on ajoute aux monomères, avant la polymérisation, 0,1 à 5,0% en poids, par rapport au polymère, d'un agent porogène à base de dioxyde de carbone, la polymérisation du mélange des monomères obtenus sous addition d'amorceurs radicalogènes ou amorçage par illumination ou irradiation sous formation d'un hydrogel, on sèche le polymère et on lui ajoute un ou plusieurs composés réactifs susceptibles de déclencher une réticulation proche de la surface subséquente, ou leur solution ou dispersion et on le chauffe ensuite à une température de 100 à 300°C.

**7.** Procédé suivant la revendication 6, caractérisé en ce que l'on sèche le polymère à des températures de 80 à 200°C, plus particulièrement de 100 à 180°C et, de préférence, de 120 à 150°C.

**8.** Procédé suivant la revendication 6, caractérisé en ce que l'on broie le polymère après le séchage.

**9.** Procédé suivant la revendication 7 ou 8, caractérisé en ce que l'on tamise le polymère jusqu'à une granulométrie de 20 à 3000 mm, de préférence de 50 à 1000 mm.

**10.** Procédé suivant la revendication 6, caractérisé en ce qu'en vue de la réticulation proche de la surface, on procède au chauffage jusqu'à une température de 100 à 300°C.

**11.** Procédé suivant la revendication 6, caractérisé en ce que l'on entreprend la polymérisation en solution aqueuse.

**12.** Procédé suivant la revendication 6, caractérisé en ce que l'on entreprend la polymérisation en une dispersion E/H.

**13.** Utilisation des polymères suivant l'une quelconque des revendications 1 à 5, à titre de composants pour des pansements pour plaies ou lésions et des articles sanitaires absorbant les liquides corporels.

**14.** Utilisation des polymères suivant l'une quelconque des revendications 1 à 5, à titre de composants dans des articles pour incontinence, des couches et des serviettes hygiéniques.

**15.** Utilisation des polymères suivant l'une quelconque des revendications 1 à 5, dans des couches avec une fraction pondérale des polymères suivant les revendications 1 à 5, par rapport à la quantité pondérale du polymère et de la bourre, de 15 à 100% en poids, de préférence 35 à 100% en poids et, bien mieux encore, de 30 à 70% en poids.

**16.** Utilisation des polymères suivant l'une quelconque des revendications 1 à 5, à titre de composants dans des câbles conducteurs de courant et de lumière.

**17.** Utilisation des polymères suivant l'une quelconque des revendications 1 à 5, à titre d'agents d'amendement du sol.

**18.** Utilisation des polymères suivant l'une quelconque des revendications 1 à 5, à titre de terre artificielle pour la culture des plantes.

**19.** Utilisation des polymères suivant l'une quelconque des revendications 1 à 5, à titre de composants dans des articles d'emballage.

**Claims**

**1.** A powdery, water-swellable, cross-linked polymer absorbing water, aqueous liquids, in particular body fluids, which is formed of:

   a) 55 - 99.9%-wt. of unsaturated, polymerizable, acid groups-containing monomers, said monomers being present as salts neutralized to the extent of at least 50 mole-%,
   b) 0 - 40%-wt. of unsaturated monomers which are copolymerizable with a),
   c) 0.1 - 5.0%-wt. of at least one cross-linking agent,
   d) 0 - 30%-wt. of a water-soluble polymer, the sum of a) - d) amounting to 100%-wt.,

characterized in that the polymer has an absorption capacity for 0.9% NaCl-solution of at least 12 g/g polymer, preferably 16 g/g polymer at a load of 60 g/cm$^2$.

2. The polymer according to claim 1 characterized in that it has an absorption rate for 0.9% NaCl-solution of at least 10 g/g polymer, preferably 16 g/g polymer, within 30 seconds.

3. The polymers according to claim 1 or 2 characterized by a particle size of 20 to 3,000 µm, preferably of 100 to 1,000 µm.

4. A powdery, water-swellable, cross-linked polymer absorbing water, aqueous liquids, in particular body fluids, which is formed of:

   a) 55 - 99.9%-wt. of unsaturated, polymerizable, acid groups-containing monomers, said monomers being present as salts neutralized to the extent of at least 50 mole-%,
   b) 0 - 40%-wt. of unsaturated monomers which are copolymerizable with a),
   c) 0.1 - 5.0%-wt. of at least one cross-linking agent,
   d) 0 - 30%-wt. of a water-soluble polymer, the sum of a) - d) amounting to 100%-wt,

   characterized in that the salts according to a) comprise as cations $Li^+$, $Na^+$, preferably $K^+$, $Cs^+$, $Rb^+$ and ammonium ions, as well as primary, secondary, and tertiary methyl ammonium ions, either alone or in combination, that 0.1 - 5.0%-wt., relative to the polymer, of a blowing agent based on carbon dioxide has been added to the monomers prior to polymerization, that the polymer has been dried and one or several compounds, or their solution or dispersion, which are reactive for secondary surface-cross-linking, have been added thereto, and that it has been heated to a temperature of 100 - $_{300°C}$.

5. The polymers according to claims 1 to 4 characterized in that the acid group-containing monomers a)
   are selected from the group consisting of acrylic acid, methacrylic acid, acrylamidomethylpropane sulfonic acid, and mixtures of these monomers,
   the monomers b)
   are selected from the group consisting of acrylamide, methacrylamide, hydroxyalkyl acrylate, dimethylaminoalkyl (meth) acrylate, dimethylaminopropyl (meth)acrylamide, the quaternary amine and ammonium salts of these monomers, and of mixtures of these monomers,
   the cross-linking agent c)
   is selected from the group consisting of alkylene bisacrylamide, N-methylol acrylamide, butanediol diacrylate, hexanediol dimethacrylate, polyglycol diacrylate, trimethylolpropane triacrylate, allyl acrylate, diallyl acrylamide, triallyl amine, diallyl ether; polyglycidyl ethers, such as ethylene glycol diglycidyl ether and glycerol polyglycidyl ether; polyols, such as glycerol; trimethylolpropane; polyalkylene glycols, such as polyethylene glycol 200 to 600; and polyamines, or mixtures of these cross-linking agents, and that the blowing agent based on carbon dioxide is a carbonate selected from the groups consisting of $Na_2CO_3$, $K_2CO_3$, $(NH_4)_2CO_3$, $MgCO_3$, $CaCO_3$, $NaHCO_3$, $KHCO_3$, mixtures of these carbonates, or carbon dioxide in gaseous or solid form.

6. A process for the production of the polymers according to claims 1 to 5 by polymerization of the monomers a), the monomers b) which are copolymerizable with a), and the cross-linking agent c) in the presence of the water-soluble polymer d), characterized in that the salts according to a) are selected from the group consisting of ions of lithium, sodium, and, preferably, of potassium, cesium, rubidium, ammonium, primary, secondary, and tertiary methyl ammonium, or of mixtures of these ions, that 0.1 - 5.0%-wt., relative to the polymer, of a blowing agent based on carbon dioxide is added to the monomers prior to polymerization; polymerization of the obtained monomer mixture under addition of radical-forming initiators or initiation by exposure to light or irradiation under formation of a hydrogel; that the polymer is dried and one or several compounds, or their solution of dispersion, which are reactive for secondary near-surface cross-linkage are added to the polymer, and that it is heated to a temperature of 100 - 300°C.

7. The process according to claim 6 characterized in that the polymer is dried at temperatures of 80 to 200°C, in particular of 100 to 180°C, and preferably of 120 to 150°C.

8. The process according to claim 6 characterized in that the polymer is ground after drying.

9. The process according to claim 7 or 8 characterized in that the polymer is screened out to a particle size of 20 - 3,000 µm, preferably of 50 - 1,000 µm.

10. The process according to claim 6 characterized in that heating to a temperature of 100 to 300°C is effected for near-surface cross-linkage.

11. The process according to claim 6 characterized in that the polymerization is conducted in aqueous solution.

12. The process according to claim 6 characterized in that the polymerization is conducted in a w/o-dispersion.

13. The use of the polymers according to claims 1 to 5 as component in sanitary articles which absorb body fluids and in wound dressings.

14. The use of the polymers according to claims 1 to 5 as component in diapers, sanitary napkins, and incontinence articles.

15. The use of the polymers according to claims 1 to 5 in diapers at a weight percentage of the polymers according to claims 1 to 5, relative to the weight amount of polymer and fluff, of 15 - 100%-wt., preferably 35 - 100%-wt., and most preferably of 30 to 70%-wt.

16. The use of the polymers according to claims 1 to 5 as component in current-conducting and light-transmitting cables.

17. The use of the polymers according to claims 1 to 5 as soil conditioner.

18. The use of the polymers according to claims 1 to 5 as artificial soil for plant breeding.

19. The use of the polymers according to claims 1 to 5 as component in packaging materials.